# EUROPÄISCHE PATENTSCHRIFT

(11) **EP 0 690 059 B1**
(45) Veröffentlichungstag und Bekanntmachung des Hinweises auf die Patenterteilung: **08.09.1999**
(21) Anmeldenummer: 95810414.3
(22) Anmeldetag: 20.06.1995
(51) Int. Cl.: C07D 487/04, C09B 57/00, C08K 5/3415

(54) **Zwei neue Kristallmodifikationen eines Diketopyrrolopyrrolpigments**
Two crystal modifications of a diketopyrrolopyrrolepigment
Deux modifications cristallines d'un pigment de dicétopyrrolopyrrole

(30) Priorität: 29.06.1994 CH 207694
(43) Veröffentlichungstag der Anmeldung: 03.01.1996
(73) Patentinhaber: Ciba Specialty Chemicals Holding Inc., 4057 Basel (CH)
(72) Erfinder: Hao, Zhimin, Dr., CH-1723 Marly (CH); Iqbal, Abul, Dr., CH-1732 Arconciel (CH); Herren, Fritz, Dr., CH-3186 Düdingen (CH)

(56) Entgegenhaltungen:
- EP-A- 0 061 426
- ACTA CRYSTALLOGRAPHICA, Bd. B48, Nr. 5, Oktober 1992 Seiten 696-700, J. MIZUGUCHI ET AL.
- ANGEWANDTE CHEMIE, Bd. 101, Nr. 4, 1989 Seiten 497-499, H. LANGHALS ET AL.

## Beschreibung

Die vorliegende Anmeldung betrifft zwei neue Kristallmodifikationen (β und γ) von 1,4-Diketo3,6-bis(3-methylphenyl)-pyrrolo[3,4-c]pyrrol, ihre Herstellung sowie die Verwendung dieser neuen Produkte als Pigmente.

Es ist allgemein bekannt, dass eine Reihe von Vertretern verschiedener organischer Pigmentklassen polymorph sind. Trotz chemisch gleicher Zusammensetzung treten solche Pigmente in zwei oder mehr Kristallmodifikationen auf. Dies ist insbesondere bei Phthalocyanin-, Chinacridon- und einigen Azopigmenten der Fall (vgl. z.B. W. Herbst, K. Hunger, Industrielle Organische Pigmente (1987), 43-45, 431-433, 458-459). Von einigen anderen Pigmenten ist hingegen nur eine einzige Kristallmodifikation bekannt. Trotz mehrerer Versuche ist es bisher z.B. nicht gelungen von irgendeinem der seit einigen Jahren bekannten und beispielsweise in den US-Patenten 4 415 685 und 4 579 949 beschriebenen Diketopyrrolopyrrolpigmente eine zweite Kristallmodifikation zu erhalten.

Vor kurzem wurde gefunden, dass Abgangsgruppen, wie z.B. solche der Formel 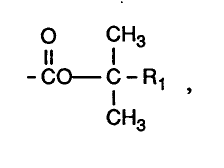 worin R₁ C₁-C₆-Alkyl ist, auch in unlösliche Stoffe, wie die Diketopyrrolopyrrolpigmente, unter Bildung von löslichen Carbamaten der Grundstruktur 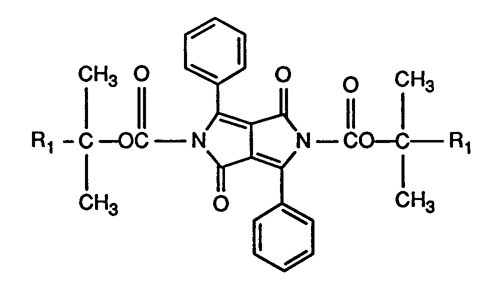 leicht eingeführt werden können und, dass durch thermische (Aufheizen auf Temperaturen zwischen 50 und 400°C), chemische (mit organischen oder anorganischen Säuren oder Basen) oder photolytische (Belichtung z.B. mit Wellenlängen unter 375 nm) Behandlung das ursprüngliche Pigment wieder zurückgebildet werden kann. Diese Untersuchungen werden in Parallelpatentanmeldungen (Anmeldedatum Oktober 1993) beschrieben.

Ganz überraschend ist nun gefunden worden, dass im Falle des Diketopyrrolopyrrols der Formel 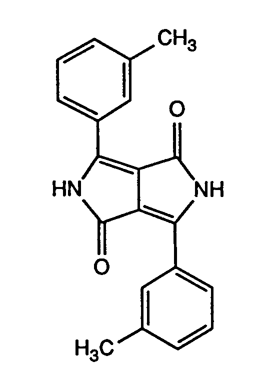 die obenerwähnte Rückbildung zum (N-unsubstituierten) Pigment durch chemische bzw. durch thermische Behandlung unter bestimmten Bedingungen nicht zur bisher bekannten, sondern je zu einer neuen Kristallmodifikation führt.

Die neue Modifikation, die durch chemische Behandlung, d.h. durch Erhitzen auf 80 bis 120°C in einem aprotischen organischen Lösungsmittel in Gegenwart einer Säure, im folgenden β-Modifikation genannt, sowie jene, die durch thermische Behandlung, d.h. durch trockenes Erhitzen auf 200 bis 350°C, im folgenden γ-Modifikation genannt, erhalten werden, unterscheiden sich voneinander und von der bekannten Modifikation, im folgenden α-Modifikation genannt, durch ein bestimmtes, verschiedenes Röntgenbeugungsdiagramm. Die β-Modifikation, die beim Erhitzen auf je nach Substrat verschieden hohe Temperaturen wieder in die α-Modifikation umgewandelt wird, zeigt überdies im Vergleich zur roten α-Modifikation eine Nuancenverschiebung gegen gelbrot, während sich die hitzestabile γ-Modifikation durch eine brillante orange Nuance auszeichnet.

Die vollständigen Röntgenbeugungsdiagramme werden nach üblichen Methoden mit Hilfe eines Siemens D500® Röntgen-Diffraktometers (CuK_{α}-Strahlung) bestimmt.

Das Röntgenbeugungsdiagramm der bekannten α-Modifikation ist durch folgende Beugungslinien

| Netzebenenabstände (d-Werte in Å) | doppelte Glanzwinkel (2 Θ) | relative Intensität |
|---|---|---|
| 13,7152 | 6,44 | 100 |
| 5,5783 | 15,88 | 46 |
| 5,1540 | 17,19 | 8 |
| 4,9920 | 17,75 | 10 |
| 4,7200 | 18,79 | 12 |
| 4,5354 | 19,56 | 30 |
| 4,3219 | 20,53 | 9 |
| 4,0783 | 21,77 | 23 |
| 3,6239 | 24,55 | 26 |
| 3,5186 | 25,29 | 22 |
| 3,3036 | 26,97 | 96 |

gekennzeichnet.

Die vorliegende Erfindung betrifft das Diketopyrrolopyrrol der Formel 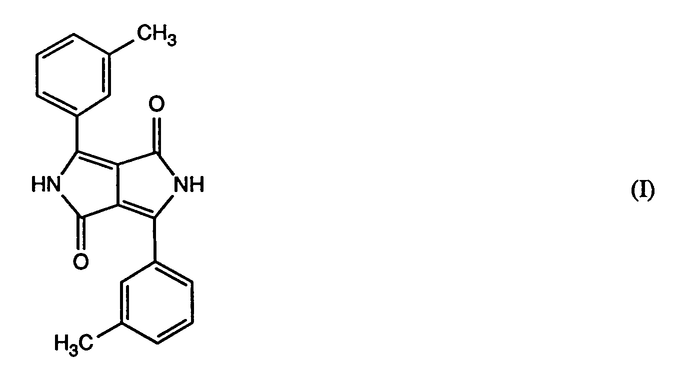
a) in seiner β-Modifikation, deren Röntgenbeugungsdiagramm durch folgende Beugungslinien

| Netzebenenabstände (d-Werte in Å) | doppelte Glanzwinkel (2 Θ) | relative Intensität |
|---|---|---|
| 13,7172 | 6,44 | 100 |
| 5,4273 | 16,32 | 44 |
| 4,7045 | 18,85 | 18 |
| 4,5517 | 19,49 | 31 |
| 4,3463 | 20,42 | 22 |
| 4,1056 | 21,63 | 11 |
| 3,4241 | 26,00 | 70 |
| 3,2844 | 27,13 | 31 |
| 3,0960 | 28,81 | 16 |
| 3,0625 | 29,14 | 18 |

gekennzeichnet ist, und
b) in seiner γ-Modifikation, deren Röntgenbeugungsdiagramm durch folgende Beugungslinien

| Netzebenenabstände (d-Werte in Å) | doppelte Glanzwinkel (2 Θ) | relative Intensität |
|---|---|---|
| 11,1386 | 7,93 | 100 |
| 6,9644 | 12,70 | 19 |
| 4,6733 | 18,98 | 10 |
| 4,3887 | 20,22 | 12 |
| 4,0033 | 22,19 | 19 |
| 3,6052 | 24,67 | 84 |
| 3,3749 | 26,39 | 12 |
| 3,2335 | 27,56 | 49 |
| 2,9053 | 30,75 | 13 |

gekennzeichnet ist.

Die γ-Modifikation ist bevorzugt.

Die Herstellung der neuen β-Modifikation erfolgt dadurch, dass ein lösliches Diketopyrrolopyrrol der Formel 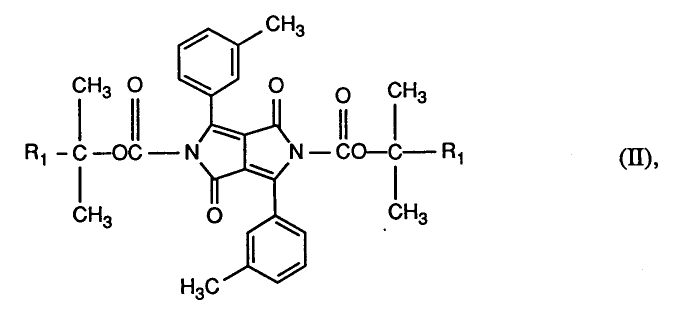 worin R₁ C₁-C₆-Alkyl ist, in einem organischen Lösungsmittel gelöst, in Gegenwart einer Säure auf einer Temperatur zwischen 80 und 120°C erhitzt wird und danach das nach dem Abkühlen ausgefallene Produkt nach üblichen Methoden isoliert wird.

Als C₁-C₆-Alkyl bedeutet R₁ z.B. Methyl, Ethyl, n-Propyl, Isopropyl, n-Butyl, tert.-Butyl, n-Amyl oder Hexyl.

Vorzugsweise bedeutet R₁ Ethyl, insbesondere aber Methyl.

Zweckmässig wird das gelöste Diketopyrrolopyrrol der Formel II in Gegenwart der Säure unter Rückfluss während 5 bis 60 Minuten, je nach Lösungsmittel, behandelt und das Gemisch danach auf 10 bis 30°C abgekühlt.

Als Lösungsmittel können inerte aprotische organische Lösungsmittel, wie z.B. Dimethylformamid, Tetrahydrofuran, Ethylenglykol, Ethylenglykolmonomethylether, Dodecan, Toluol, Xylol, Acetylaceton, Dimethylsulfoxid oder deren Mischungen verwendet werden. Bevorzugt werden Dimethylsulfoxid, Acetylaceton, Ethylenglykolmonomethylether und insbesondere Dimethylformamid.

Als Säuren eignen sich sowohl anorganische, als auch organische Säuren, wie z.B. Salzsäure, Schwefelsäure, Toluolsulfonsäure oder Trifluoressigsäure. Toluol-4-sulfonsäure ist bevorzugt. Zweckmässig werden 8 bis 30, bevorzugt 15 bis 20 Mol Säure auf 1 Mol Diketopyrrolopyrrol der Formel II eingesetzt. Die Säure kann entweder vor, zusammen, mit oder nach der Pigmentsalzsuspension zugegeben werden, vorzugsweise vor oder zusammen mit der Pigmentsalzsuspension.

Bevorzugt verwendet man 15 bis 20 Mol Toluol-4-sulfonsäure, bezogen auf das Diketo-pyrrolopyrrol, in N,N-Dimethylformamid bei 100 bis 105°C während 15 bis 20 Minuten.

Die Herstellung der neuen γ-Modifikation erfolgt dadurch, dass ein Diketopyrrolopyrrol der Formel 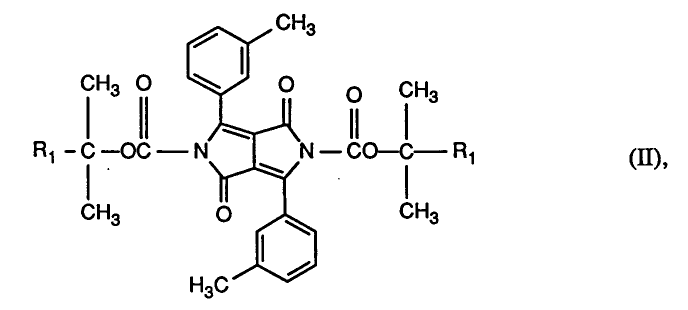 worin R₁ C₁-C₆-Alkyl ist, während 10 Minuten bis 10 Stunden auf einer Temperatur von 200 bis 350°C erhitzt wird.

Vorzugsweise wird das Diketopyrrolopyrrol der Formel II in Pulverform 30 Minuten bis 2 Stunden auf 220 bis 260°C erhitzt.

Diketopyrrolopyrrole der Formel II können in Analogie zu allgemein bekannten Methoden z.B. durch Umsetzung eines Diketopyrrolopyrrols der Formel I mit einem Dicarbonat der Formel 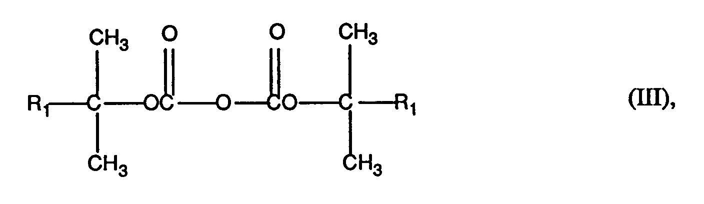 oder mit einem Trihaloessigsäureester der Formel 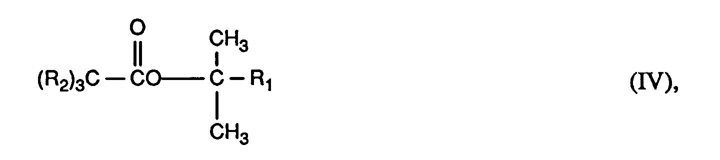 worin R₂ Chlor, Fluor oder Brom bedeutet,
oder mit einem Azid der Formel 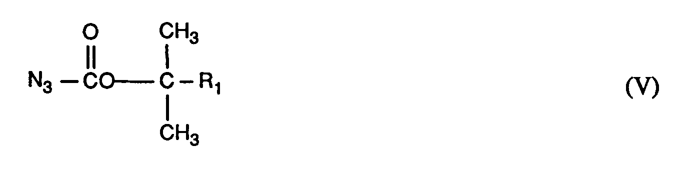 wobei R₁ jeweils die oben angegebene Bedeutung hat,
in einem aprotischen organischen Lösungsmittel in Gegenwart einer Base als Katalysator, bei Temperaturen zwischen 0 und 400°C während 2 bis 80 Stunden, erhalten werden.

Das Dicarbonat der Formel III, der Trihaloessigsäureester der Formel IV oder das Azid der Formel V wird zweckmässig in einem 2- bis 10-fachem Überschuss eingesetzt.

Bevorzugt wird das Diketopyrrolopyrrol der Formel I mit einem Dicarbonat der Formel III umgesetzt.

Dicarbonate der Formel III, Trihaloessigsäureester der Formel IV und Azide der Formel V sind bekannte Substanzen. Sollten einige noch neu sein, so können sie in Analogie zu allgemein bekannten Methoden hergestellt werden.

Als Lösungsmittel eignen sich beispielsweise Ether, wie Tetrahydrofuran oder Dioxan, oder Glykoläther, wie Ethylenglykol-methyläther, Ethylenglykol-ethylether, Diethylenglykol-monomethylether oder Diethylenglykol-monoethylether, ferner dipolar-aprotische Lösungsmittel, wie Acetonitril, Benzonitril, N,N-Dimethylformamid, N,N-Dimethylacetamid, Nitrobenzol, N-Methylpyrrolidon, halogenierte aliphatische oder aromatische Kohlenwasserstoffe, wie Trichlorethan, Benzol oder durch Alkyl, Alkoxy oder Halogen substituiertes Benzol, wie Toluol, Xylol, Anisol oder Chlorbenzol oder aromatische N-Heterocyclen, wie Pyridin, Picolin oder Chinolin. Bevorzugte Lösungsmittel sind z.B. Tetrahydrofuran, N,N-Dimethylformamid, N-Methylpyrrolidon. Die genannten Lösungsmittel können auch als Mischungen eingesetzt werden. Zweckmässigerweise verwendet man 5-20 Gew.-Teile Lösungsmittel auf 1 Gew.-Teil der Reaktionsteilnehmer.

Als Katalysator geeignete Basen sind beispielsweise die Alkalimetalle selbst, wie Lithium, Natrium oder Kalium sowie deren Hydroxyde oder Carbonate, oder Alkaliamide, wie Lithium-, Natrium- oder Kaliumamid oder Alkalihydride, wie Lithium-, Natrium-oder Kaliumhydrid, oder Erdalkali- oder Alkalialkoholate, die sich insbesondere von primären, sekundären oder tertiären aliphatischen Alkoholen mit 1 bis 10 C-Atomen ableiten, wie z.B. Lithium-, Natrium- oder Kaliummethylat, -ethylat, -n-propylat, -isopropylat, n-butylat, -sek.-butylat, -tert.-butylat, -2-methyl-2-butylat, -2-methyl-2-pentylat, -3-methyl-3-pentylat, -3-ethyl-3-pentylat, und ferner organische aliphatische, aromatische oder heterocyclische N-Basen, darunter z.B. Diazabicyclooctan, Diazabicycloundecen und 4-Dimethylaminopyridin und Trialkylamine, wie z.B. Trimethyl- oder Triethylamin. Man kann aber auch ein Gemisch der genannten Basen verwenden.

Bevorzugt werden die organischen N-Basen, wie z.B. Diazabicyclooctan, Diazabicyclo-undecen und insbesondere 4-Dimethylaminopyridin.

Die Umsetzung wird bevorzugt bei Temperaturen zwischen 10 und 100°C, insbesondere zwischen 14 und 40°C, durchgeführt und zwar bei atmosphärischem Druck.

Auch die erfindungsgemässen β- und γ-Diketopyrrolopyrrole eignen sich, wie bereits z.B. in den US-Patenten 4 415 685 und 4 579 949 für deren α-Modifikation beschrieben, als Pigmente zum Färben von hochmolekularem organischem Material. Da das β-Diketopyrrolopyrrol aber beim Erhitzen auf je nach Substrat verschieden hohe Temperaturen wieder in die α-Modifikation umgewandelt wird, ist bei einer Verwendung in Materialien, die bei höheren Temperaturen verarbeitet werden, Vorsicht geboten. Das γ-Diketopyrrolopyrrol ist hingegen sehr stabil und eignet sich sehr gut für die Anwendung selbst in hochmolekularen Materialien, wie z.B. Polyolefine und Polyester, mit sehr hoher Sättigung.

Wie viele andere Pigmente, können die erfindungsgemässen β- und γ-Diketopyrrolopyrrole auch mit Vorteil nach bekannten Methoden oberflächenbehandelt werden, um ihre Eigenschaften in Lacksystemen zu verbessern. Additive, die zur Verminderung oder Vermeidung von Flokkulation, sowie zur Verbesserung der Dispersionsstabilität dienen, können vorteilhaft mit den erfindungsgemässen Pigmenten verwendet werden. Die so behandelten Pigmente zeigen gute Eigenschaften allein oder in Mischung mit anderen Pigmenten zur Erzeugung von roten bis orangen Volltonausfärbungen in verschiedenen Lacksystemen, jedoch bevorzugt in Automobillacksystemen des Acryl-, Alkyd-und Polyestertyps. 2-Phthalimidomethylchinacridon, Chinacridonsulfonsäure und andere ähnliche Derivate können z.B. als Antiflokkulationsmittel dienen. In bestimmten Systemen kann der Zusatz von polymeren Dispergiermittel die Eigenschaften der Pigmente noch zusätzlich verbessern.

Die erfindungsgemässen β- und γ-Diketopyrrolopyrrole werden in Mengen von 0,01 bis 30 Gew.-%, bevorzugt 0,1 bis 10 Gew.-% bezogen auf das zu färbende hochmolekulare organische Material eingesetzt und in diesem zweckmässig bei Temperaturen zwischen 20 und 180°C für die β-Form und zwischen 20 und 300°C für das γ-Diketopyrrolopyrrol eingearbeitet.

Die erfindungsgemässen β- und γ-Diketopyrrolopyrrole lassen sich beispielsweise als Pulver, Teig, Flushpaste und Zubereitung anwenden und eignen sich z.B. für Druckfarben, Leimfarben, Binderfarben oder Lacke aller Art, wie physikalisch und oxydativ trocknende Lacke, säure-, amin- und peroxid-härtende Lacke oder Polyurethanlacke. Die erfindungsgemässen Pigmente können, je nach ihrer Verträglichkeit mit der Verarbeitungstemperatur, auch zum Färben von synthetischen, halbsynthetischen oder natürlichen makromolekularen Stoffen verwendet werden, wie Polyvinylchlorid, Polystyrol, Polyethylen, Polypropylen, Polyestern, Phenoplasten, Aminoplasten und Gummi. Weitere Anwendungsbeispiele sind das Färben von natürlichen, regenerierten oder künstlichen Fasern, wie Glas-, Silikat-, Asbest-, Holz-, Cellulose-, Acetylcellulose-, Polyacrylnitril-, Polyester-, Polyurethan- und Polyvinylchloridfasern oder deren Gemischen, eventuell zusammen mit anderen organischen oder anorganischen Pigmenten. Die erhaltenen Färbungen, beispielsweise in Lacken, Drucken oder Kunststoffen, zeichnen sich durch einen gelbroten bis orangen Farbton, gute Überlackier-, Migrations-, Licht-und Wetterechtheit sowie durch hohe Farbstärke und Transparenz aus.

Die erfindungsgemässen Pigmente können zum Färben von festen, elastischen, pastenartigen, dickflüssigen, dünnflüssigen oder thixotropen Massen verwendet und in diese nach an sich bekannten Verfahren eingearbeitet werden. Wasserhaltige Teige können beispielsweise durch Einrühren des Pigments in Wasser, gegebenenfalls unter Zusatz eines Netz- oder Dispergiermittels oder durch Einrühren oder Einkneten des Pigments in ein Dispergiermittel in Gegenwart von Wasser und gegebenenfalls von organischen Lösungsmitteln oder Ölen erhalten werden. Diese Teige können beispielsweise wiederum zur Herstellung von Flushpasten, Druckfarben, Leimfarben und Kunststoffdispersionen verwendet werden. Das Pigment kann aber auch durch Einrühren, Einwalzen, Einkneten oder Einmahlen in Wasser, organische Lösungsmittel, nicht trocknende Öle, trocknende Öle, Lacke, Kunststoffe oder Gummi gebracht werden. Schliesslich ist es auch möglich, das Pigment durch trockenes Mischen mit organischen oder anorganischen Massen, Granulaten, Faserstoffen, Pulvern und anderen Pigmenten zu Stoffmischungen zu verarbeiten.

Die nachfolgenden Beispiele erläutern die Erfindung.

### Beispiel 1a: (Herstellung des löslichen Diketopyrrolopyrrols)

Einer Mischung von 10,0 g 1,4-Diketo-3,6-di-(3-methylphenyl)-pyrrolo[3,4-c]pyrrol und 1,0 g 4-Dimethylaminopyridin in 350 ml Tetrahydrofuran (über Molekularsieb getrocknet) werden 15,2 g Di-tert.-butyldicarbonat zugegeben. Die erhaltene orange Suspension wird 20 Stunden bei Raumtemperatur unter Ausschluss von atmosphärischer Feuchtigkeit gerührt. Danach wird das Lösungsmittel bei vermindertem Druck abdestilliert. Der braune Rückstand wird zuerst mit Wasser, dann mit Methanol gewaschen und im Vakuum bei Raumtemperatur getrocknet. Man erhält 14,1 g (86,5 % d.Th.) eines leuchtend gelben Produktes der Formel

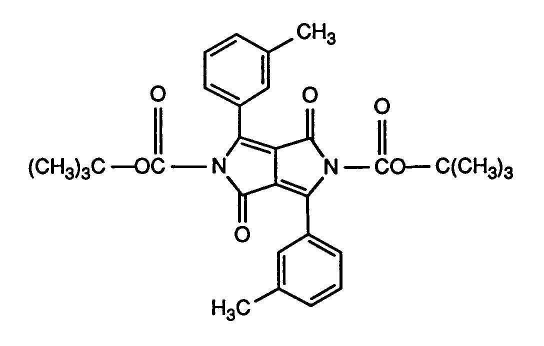

| Analyse: | C | H | N |
|---|---|---|---|
| Ber.: | 69,75 % | 6,24 % | 5,42 % |
| Gef.: | 69,82 % | 6,40 % | 5,47 % |

b) Einer Lösung von 1,0 g des Produktes aus a) in 75 ml N,N-Dimethylformamid werden 7,4 g Toluol-4-sulfonsäure zugegeben. Die Mischung wird auf 100°C geheizt, 15 Minuten bei dieser Temperatur gerührt und anschliessend im Eisbad auf 20°C abgeschreckt. Das ausgefallene Pigment wird abfiltriert, mit Methanol und dann mit Wasser gewaschen und im Vakuum bei 60°C getrocknet. Man erhält 0,43 g eines roten Pulvers.

| Analyse: | C | H | N |
|---|---|---|---|
| Ber.: | 75,93 % | 5,10 % | 8,86 % |
| Gef.: | 75,91 % | 5,14 % | 8,69 % |

Das Röntgenbeugungsdiagramm ist durch folgende Beugungslinien

| Netzebenenabstände (d-Werte in Å) | doppelte Glanzwinkel (2 Θ) | relative Intensität |
|---|---|---|
| 13,7172 | 6,44 | 100 |
| 5,4273 | 16,32 | 44 |
| 4,7045 | 18,85 | 18 |
| 4,5517 | 19,49 | 31 |
| 4,3463 | 20,42 | 22 |
| 4,1056 | 21,63 | 11 |
| 3,4241 | 26,00 | 70 |
| 3,2844 | 27,13 | 31 |
| 3,0960 | 28,81 | 16 |
| 3,0625 | 29,14 | 18 |

gekennzeichnet.

### Beispiel 2:

2,3 g des Produktes aus Beispiel la werden in eine Kristallisierschale eingebracht, dann in einen auf 240°C vorgeheizten Ofen eingeführt und eine Stunde bei dieser Temperatur gehalten. Danach wird das Produkt auf Raumtemperatur abkühlen gelassen. Man erhält 1,3 g eines orangen Pulvers, welches in PVC eingearbeitet eine brillante orange Färbung ergibt.

| Analyse: | C | H | N |
|---|---|---|---|
| Ber.: | 75,93 % | 5,10 % | 8,86 % |
| Gef.: | 75,75 % | 5,09 % | 8,86 % |

Das Röntgenbeugungsdiagramm ist durch folgende Beugungslinien

| Netzebenenabstände (d-Werte in Å) | doppelte Glanzwinkel (2 Θ) | relative Intensität |
|---|---|---|
| 11,1386 | 7,93 | 100 |
| 6,9644 | 12,70 | 19 |
| 4,6733 | 18,98 | 10 |
| 4,3887 | 20,22 | 12 |
| 4,0033 | 22,19 | 19 |
| 3,6052 | 24,67 | 84 |
| 3,3749 | 26,39 | 12 |
| 3,2335 | 27,56 | 49 |
| 2,9053 | 30,75 | 13 |

gekennzeichnet.

### Beispiel 3:

7,5 g des Pigments, dessen Herstellung in Beispiel 1b beschrieben wird, 98,9 g CAB-Lösung bestehend aus
- 41,00 g: Celluloseacetobutyrat ®CAB 531.1, 20 %ig in Butanol/Xylol 2:1 (Eastman Chem.)
- 1,50 g: Zirkonium Octoat
- 18,50 g: ®SOLVESSO 150*
- 21,50 g: Butylacetat und
- 17,50 g: Xylol
36,50 g Polyesterharz ®DINAPOL H700 (Dynamit Nobel), 4,60 g Melaminharz ®MAPRENAL MF650 (Hoechst) und 2,50 g Dispergiermittel ®DISPERBYK 160 (Byk Chemie) werden zusammen während 90 Minuten mit einer Schüttelmaschine dispergiert (Total Lack 150 g, 5 % Pigment).

27,69 g des so erhaltenen Volltonlacks werden für die Base-coat-Lackierung mit 17,31 g Al-Stammiösung (8 %ig) bestehend aus
- 12,65 g: ®SILBERLINE SS 3334AR, 60 %ig (Silberline Ltd.)
- 56,33 g: CAB Lösung (Zusammensetzung wie oben)
- 20,81 g: Polyesterharz ®DINAPOL H700
- 2,60 g: Melaminharz ®MAPRENAL MF650
- 7,59 g: ®SOLVESSO 150* *
gemischt und auf ein Aluminiumblech spritzappliziert (Nassfilm ca. 20 µm). Nach einer Abdunstzeit von 30 Minuten bei Raumtemperatur wird ein TSA-Lack bestehend aus
- 29,60 g: Acrylharz ®URACRON 2263 XB, 50 %ig in Xylol/Butanol (Chem. Fabrik Schweizerhalle),
- 5,80 g: Melaminharz ®CYMEL 327,90 %ig in Isobutanol,
- 2,75 g: Butylglycolacetat,
- 5,70 g: Xylol,
- 1,65 g: n-Butanol,
- 0,50 g: Siliconöl, 1 %ig in Xylol,
- 3,00 g: Lichtschutzmittel ®TINUVIN 900, 10 %ig in Xylol (Ciba),
- 1,00 g: Lichtschutzmittel ®TINUVIN 292, 10 %ig in Xylol (Ciba)
als Top-coat-Lackierung spritzappliziert (Nassfilm ca. 50 µm). Anschliessend wird der Lack nach weiteren 30 Minuten Abdunsten bei Raumtemperatur, 30 Minuten bei 130°C eingebrannt.
* ®SOLVESSO 150 = aromatisches Lösungsmittel von ESSO
* ®SOLVESSO 150 = aromatisches Lösungsmittel von ESSO

### Beispiel 4:

0,6 g des gemäss Beispiel 1b hergestellten Pigments werden mit 67 g Polyvinylchlorid, 33 g Dioctylphthalat, 2 g Dibutylzinndilaurat und 2 g Titandioxid vermischt und auf einem Walzenstuhl während 15 Minuten bei 160°C zu einer dünnen Folie verarbeitet. Die so erzeugte rote PVC-Folie zeichnet sich durch sehr gute Echtheiten aus.

### Beispiel 5:

Ersetzt man das im Beispiel 3 verwendete Pigment durch die gleiche Menge des gemäss Beispiel 2 hergestellten Pigments, so erhält man nach Aufarbeitung gemäss Beispiel 3 eine hochgesättigte orange Lackfärbung mit ausgezeichneten Echtheiten.

### Beispiel 6:

Ersetzt man das im Beispiel 4 verwendete Pigment durch die gleiche Menge des gemäss Beispiel 2 hergestellten Pigments, so erhält man nach Aufarbeitung gemäss Beispiel 4 eine hochgesättigte orange PVC-Färbung mit ausgezeichneten Echtheiten.

## Patentansprüche

1. Diketopyrrolopyrrol der Formel 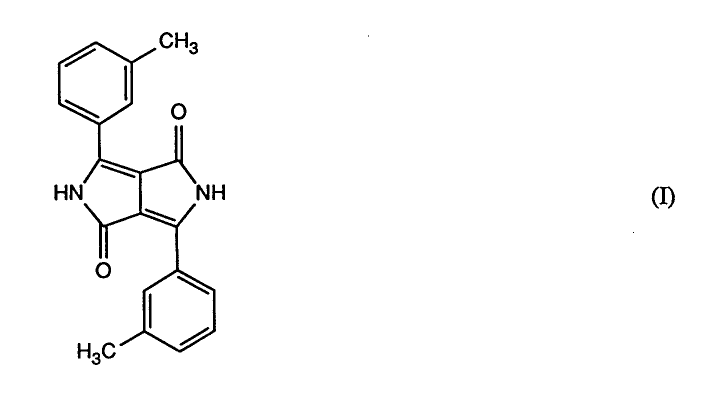
a) in seiner β-Modifikation, deren Röntgenbeugungsdiagramm durch folgende Beugungslinien
| Netzebenenabstände (d-Werte in Å) | doppelte Glanzwinkel (2 Θ) | relative Intensität |
|---|---|---|
| 13,7172 | 6,44 | 100 |
| 5,4273 | 16,32 | 44 |
| 4,7045 | 18,85 | 18 |
| 4,5517 | 19,49 | 31 |
| 4,3463 | 20,42 | 22 |
| 4,1056 | 21,63 | 11 |
| 3,4241 | 26,00 | 70 |
| 3,2844 | 27,13 | 31 |
| 3,0960 | 28,81 | 16 |
| 3,0625 | 29,14 | 18 |
gekennzeichnet ist, und
b) in seiner γ-Modifikation, deren Röntgenbeugungsdiagramm durch folgende Beugungslinien
| Netzebenenabstände (d-Werte in Å) | doppelte Glanzwinkel (2 Θ) | relative Intensität |
|---|---|---|
| 11,1386 | 7,93 | 100 |
| 6,9644 | 12,70 | 19 |
| 4,6733 | 18,98 | 10 |
| 4,3887 | 20,22 | 12 |
| 4,0033 | 22,19 | 19 |
| 3,6052 | 24,67 | 84 |
| 3,3749 | 26,39 | 12 |
| 3,2335 | 27,56 | 49 |
| 2,9053 | 30,75 | 13 |
gekennzeichnet ist.

2. Diketopyrrolopyrrol gemäss Anspruch 1 in seiner γ-Modifikation.

3. Verfahren zur Herstellung des Diketopyrrolopyrrols der Formel 1 gemäss Anspruch 1 in seiner β-Modifikation, dadurch gekennzeichnet, dass ein lösliches Diketopyrrolopyrrol der Formel 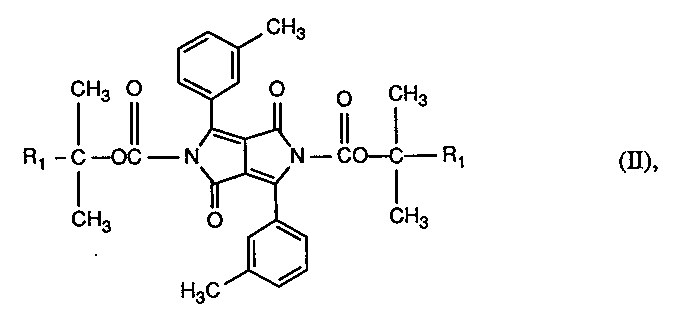 worin R₁ C₁-C₆-Alkyl ist, in einem organischen Lösungsmittel gelöst, in Gegenwart einer Säure auf einer Temperatur zwischen 80 und 120°C erhitzt wird und danach das nach dem Abkühlen ausgefallene Produkt nach üblichen Methoden isoliert wird.

4. Verfahren gemäss Anspruch 3, dadurch gekennzeichnet, dass eine Verbindung der Formel II verwendet wird, worin R₁ Ethyl oder bevorzugt Methyl ist.

5. Verfahren gemäss Anspruch 3, dadurch gekennzeichnet, dass das Diketopyrrolopyrrol der Formel II unter Rückfluss während 5 bis 60 Minuten behandelt und das Gemisch danach auf 10 bis 30°C abgekühlt wird.

6. Verfahren gemäss Anspruch 3, dadurch gekennzeichnet, dass als Lösungsmittel Dimethylsulfoxid, Acetylaceton, Ethylenglykolmonomethylether oder bevorzugt Dimethylformamid verwendet wird.

7. Verfahren gemäss Anspruch 3, dadurch gekennzeichnet, dass als Säure Toluol-4-sulfonsäure verwendet wird.

8. Verfahren gemäss Anspruch 3, dadurch gekennzeichnet, dass 8 bis 30 Mol Säure auf 1 Mol Diketopyrrolopyrrol der Formel II eingesetzt werden.

9. Verfahren gemäss Anspruch 3, dadurch gekennzeichnet, dass das Diketopyrrolopyrrol der Formel II mit 15 bis 20 Mol Toluol-4-sulfonsäure, bezogen auf das Diketopyrrolopyrrol 15 bis 20 Minuten bei 100-105°C in Dimethylformamid behandelt wird.

10. Verfahren zur Herstellung des Diketopyrrolopyrrols der Formel I gemäss Anspruch 1 in seiner γ-Modifikation dadurch gekennzeichnet, dass ein Diketopyrrolopyrrol der Formel 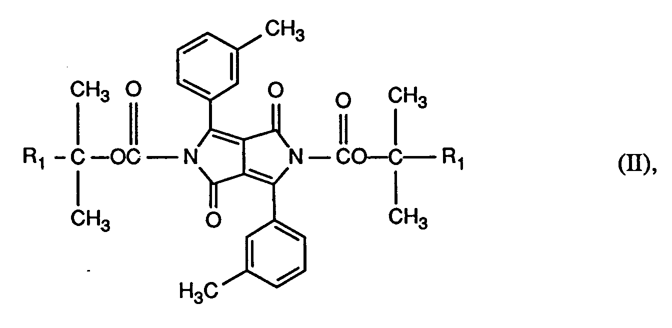 worin R₁ C₁-C₆-Alkyl ist, während 10 Minuten bis 10 Stunden auf einer Temperatur von 200 bis 350°C erhitzt wird.

11. Verfahren gemäss Anspruch 10, dadurch gekennzeichnet, dass das Diketopyrrolopyrrol der Formel II in Pulverform 30 Minuten bis 2 Stunden auf 220 bis 260°C erhitzt wird.

12. Hochmolekulares organisches Material enthaltend ein Diketopyrrolopyrrol gemäss Anspruch 1.

13. Hochmolekulares organisches Material gemäss Anspruch 12, enthaltend das Diketopyrrolopyrrol gemäss Anspruch 1 in seiner γ-Modifikation.

## Claims

1. A diketopyrrolopyrrole of the formula 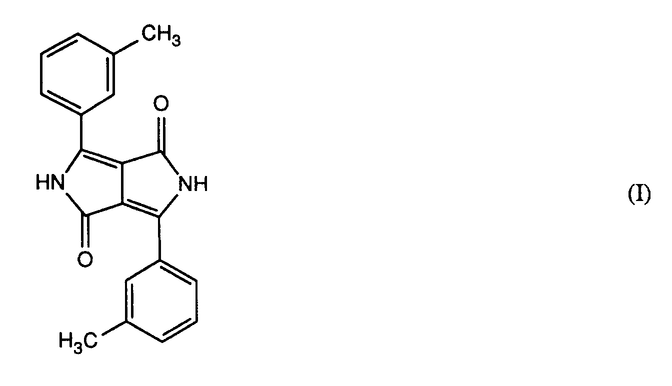
a) in its β-modification, whose X-ray diffraction diagram is characterized by the following diffraction lines
| interplanar spacings (d values in Å) | twice the Bragg angle (2 Θ) | relative intensity |
|---|---|---|
| 13.7172 | 6.44 | 100 |
| 5.4273 | 16.32 | 44 |
| 4.7045 | 18.85 | 18 |
| 4.5517 | 19.49 | 31 |
| 4.3463 | 20.42 | 22 |
| 4.1056 | 21.63 | 11 |
| 3.4241 | 26.00 | 70 |
| 3.2844 | 27.13 | 31 |
| 3.0960 | 28.81 | 16 |
| 3.0625 | 29.14 | 18 |
or
b) in its γ-modification, whose X-ray diffraction diagram is characterized by the following diffraction lines
| interplanar spacings (d values in Å) | twice the Bragg angle (2 Θ) | relative intensity |
|---|---|---|
| 11.1386 | 7.93 | 100 |
| 6.9644 | 12.70 | 19 |
| 4.6733 | 18.98 | 10 |
| 4.3887 | 20.22 | 12 |
| 4.0033 | 22.19 | 19 |
| 3.6052 | 24.67 | 84 |
| 3.3749 | 26.39 | 12 |
| 3.2335 | 27.56 | 49 |
| 2.9053 | 30.75 | 13. |

2. A diketopyrrolopyrrole according to claim 1 in its γ-modification.

3. A process for the preparation of a diketopyrrolopyrrole of the formula 1 according to claim 1 in its β-modification, which comprises dissolving a soluble diketopyrrolopyrrole of the formula 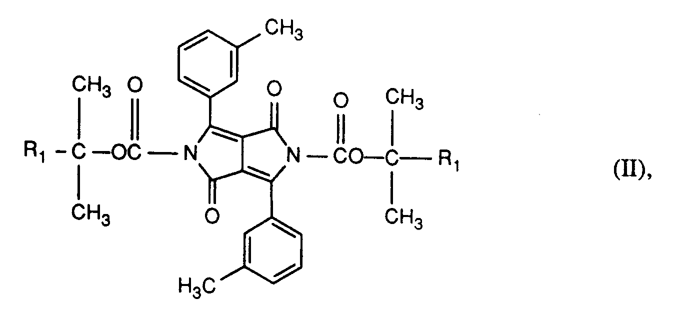 in which R₁ is C₁-C₆alkyl, in an organic solvent, heating the solution at a temperature of between 80 and 120°C in the presence of an acid, and then isolating the product, which has precipitated after cooling, by conventional methods.

4. A process according to claim 3, wherein a compound of the formula II is used in which R₁ is ethyl or, preferably, methyl.

5. A process according to claim 3, wherein the diketopyrrolopyrrole of the formula II is treated under reflux for from 5 to 60 minutes and the mixture is then cooled to from 10 to 30°C.

6. A process according to claim 3, wherein the solvent used is dimethyl sulfoxide, acetylacetone, ethylene glycol monomethyl ether or, preferably, dimethylformamide.

7. A process according to claim 3, wherein the acid used is 4-toluenesulfonic acid.

8. A process according to claim 3, wherein from 8 to 30 mol of acid are employed per mole of diketopyrrolopyrrole of the formula II.

9. A process according to claim 3, wherein the diketopyrrolopyrrole of the formula II is treated with from 15 to 20 mol of 4-toluenesulfonic acid, based on the diketopyrrolopyrrole, in dimethylformamide at 100-105°C for from 15 to 20 minutes.

10. A process for the preparation of the diketopyrrolopyrrole of the formula I according to claim 1 in its γ-modification, which comprises heating a diketopyrrolopyrrole of the formula 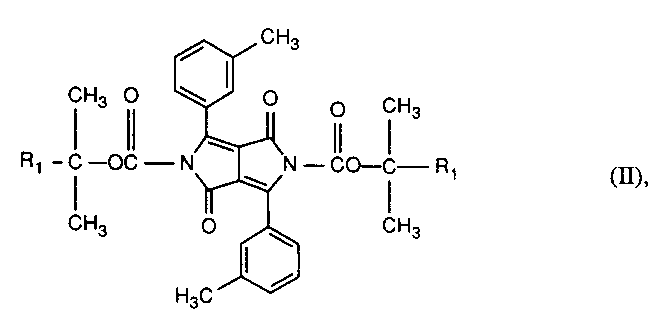 in which R₁ is C₁-C₆alkyl, at a temperature of from 200 to 350°C for from 10 minutes to 10 hours.

11. A process according to claim 10, wherein the diketopyrrolopyrrole of the formula II is heated in powder form at from 220 to 260°C for from 30 minutes to 2 hours.

12. A high molecular weight organic material comprising a diketopyrrolopyrrole according to claim 1.

13. A high molecular weight organic material according to claim 12, comprising the dlketopyrrolopyrrole according to claim 1 in its γ-modification.

## Revendications

1. Dicétopyrrolopyrrole de formule 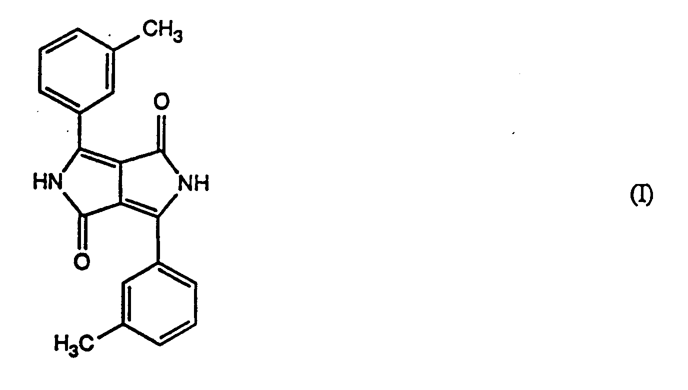
a) dans sa modification β, dont le diagramme de diffraction des rayons X est caractérisé par les lignes de diffraction suivantes
| Distance entre les plans réticulaires (valeurs de d en Å) | Double de l'angle de Bragg (2 Θ) | Intensité relative |
|---|---|---|
| 13,7172 | 6,44 | 100 |
| 5,4273 | 16,32 | 44 |
| 4,7045 | 18,85 | 18 |
| 4,5517 | 19,49 | 31 |
| 4,3463 | 20,42 | 22 |
| 4,1056 | 21,63 | 11 |
| 3,4241 | 26,00 | 70 |
| 3,2844 | 27,13 | 31 |
| 3,0960 | 28,81 | 16 |
| 3,0625 | 29,14 | 18 |
b) dans sa modification γ, dont le diagramme de diffraction des rayons X est caractérisé par les lignes de diffraction suivantes
| Distance entre les plans réticulaires (valeurs de d en Å) | Double de l'angle de Bragg (2 Θ) | Intensité relative |
|---|---|---|
| 11,1386 | 7,93 | 100 |
| 6,9644 | 12,70 | 19 |
| 4,6733 | 18,98 | 10 |
| 4,3887 | 20,22 | 12 |
| 4,0033 | 22,19 | 19 |
| 3,6052 | 24,67 | 84 |
| 3,3749 | 26,39 | 12 |
| 3,2335 | 27,56 | 49 |
| 2,9053 | 30,75 | 13 |

2. Dicétopyrrolopyrrole selon la revendication 1 dans sa modification γ.

3. Procédé de préparation du dicétopyrrolopyrrole selon la revendication 1 dans sa modification β, caractérisé en ce qu'on dissout un dicétopyrrolopyrrole soluble de formule 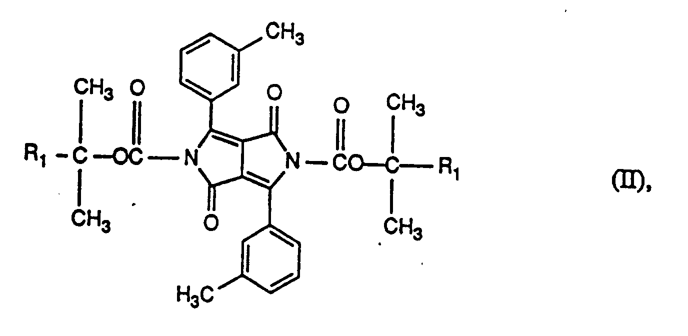 dans laquelle R₁ est un groupe alkyle en C₁-C₆, dans un solvant organique, en ce qu'on le chauffe en présence d'un acide à une température entre 80 et 120°C et on isole ensuite le produit précipité après le refroidissement selon des procédés usuels.

4. Procédé selon la revendication 3, caractérisé en ce qu'on utilise un composé de formule II, dans laquelle R₁ est un groupe éthyle ou de préférence méthyle.

5. Procédé selon la revendication 3, caractérisé en ce qu'on traite le dicétopyrrolopyrrole de formule II à reflux pendant 5 à 60 minutes et on refroidit ensuite le mélange à 10 à 30°C.

6. Procédé selon la revendication 3, caractérisé en ce qu'on utilise comme solvant le diméthylsulfoxyde, l'acétylacétone, l'éther monométhylique d'éthylèneglycol ou de préférence le diméthylformamide.

7. Procédé selon la revendication 3, caractérisé en ce qu'on utilise comme acide l'acide toluène-4-sulfonique.

8. Procédé selon la revendication 3, caractérisé en ce qu'on utilise 8 à 30 moles d'acide pour 1 mole de dicétopyrrolopyrrole de formule II.

9. Procédé selon la revendication 3, caractérisé en ce qu'on traite le dicétopyrrolopyrrole de formule II avec 15 à 20 moles d'acide toluène-4-sulfonique, par rapport au dicétopyrrolopyrrole, 15 à 20 minutes à 100 - 105°C dans le diméthylformamide.

10. Procédé de préparation du dicétopyrrolopyrrole de formule I selon la revendication 1 dans sa modification γ, caractérisé en ce qu'on chauffe un dicétopyrrolopyrrole de formule 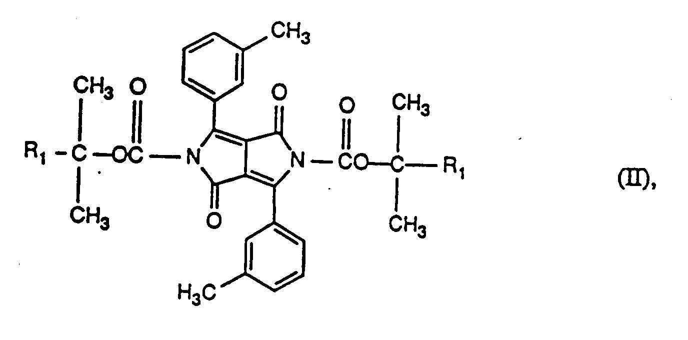 dans laquelle R₁ est un groupe alkyle en C₁-C₆, pendant 10 minutes à 10 heures à une température de 200 à 350°C.

11. Procédé selon la revendication 10, caractérisé en ce qu'on chauffe le dicétopyrrolopyrrole de formule II sous forme de poudre 30 minutes à 2 heures à 220 à 260°C.

12. Matériau organique de poids moléculaire élevé contenant un dicétopyrrolopyrrole selon la revendication 1.

13. Matériau organique de poids moléculaire élevé selon la revendication 12, contenant le dicétopyrrolopyrrole selon la revendication 1 dans sa modification γ.
